# EUROPÄISCHE PATENTSCHRIFT

(11) **EP 3 172 977 B1**
(45) Veröffentlichungstag und Bekanntmachung des Hinweises auf die Patenterteilung: **03.01.2018**
(21) Anmeldenummer: 15020237.2
(22) Anmeldetag: 27.11.2015
(51) Int. Cl.: B32B 37/14, B32B 38/00, A41C 5/00, A61F 13/00, A41C 3/00

(54) **VERFAHREN ZUR HERSTELLUNG VON BEKLEIDUNGSSTÜCKEN ODER BANDAGEN**
METHOD FOR FORMING GARMENTS OR BANDAGES
PROCÉDÉ DE FABRICATION D'ARTICLES VESTIMENTAIRES OU DE BANDAGES

(43) Veröffentlichungstag der Anmeldung: 31.05.2017
(73) Patentinhaber: NTT New Textile Technologies GmbH, 72336 Balingen (DE)
(72) Erfinder: Bauer, Hans, D-72336 Balingen (DE)
(74) Vertreter: Müller, Gottfried

(56) Entgegenhaltungen:
- WO-A2-2012/139758
- DE-A1-102012 101 937
- US-A- 3 930 090

## Beschreibung

Die Erfindung bezieht sich auf ein Verfahren zur Herstellung von Bekleidungsstücken oder Bandagen.

Aus der DE 101 33 644 C2 ist ein Verfahren zur Herstellung eines Unterbekleidungs- oder Sportbekleidungsstücks bekannt, bei dem in einem ersten Verfahrensschritt auf eine Stofflage eine Elastomer-Klebebahn aufgetragen wird, die der späteren Außenkontur des herzustellenden Bekleidungsstückes entspricht. In einem zweiten Verfahrensschritt wird das Gewebe im Bereich der Elastomer-Klebebahn ausgeschnitten.

Verschiedene Abschnitte eines Bekleidungsstücks können unterschiedlich hohen Belastungen unterliegen, was bei der Wahl der Stofflage zu berücksichtigen ist. Zugleich ist jedoch insbesondere bei Unterbekleidungsstücken auf einen hohen Tragekomfort zu achten.

Aus der DE 10 2012 101 937 A1 ist ein Verfahren zur Herstellung von Bandagen bekannt, bei dem ein Elastomer im noch nicht abgebundenen Zustand auf eine elastische Stofflage aufgebracht wird. Zur Verstärkung und Versteifung können in das Elastomer Verstärkungselemente eingebettet sein, die beim Herstellungsprozess von dem Elastomermaterial umspritzt werden.

Nach dem Aushärten sind die Verstärkungselemente vollständig von dem Elastomer eingehüllt.

Zum weiteren Stand der Technik wird auf die Druckschriften US 2002/0 106 970 A1, EP 0187 270 A1, US 3 930 090 A und WO 2012/139758 A2 verwiesen.

Der Erfindung liegt die Aufgabe zugrunde, mit einfachen Maßnahmen ein Bekleidungsstück oder eine Bandage herzustellen, das bzw. die bei hohem Tragekomfort auch höheren Belastungen standhält.

Diese Aufgabe wird erfindungsgemäß mit den Merkmalen des Anspruches 1 gelöst. Die Unteransprüche geben zweckmäßige Weiterbildungen an.

Das erfindungsgemäße Verfahren bezieht sich auf die Herstellung von Bekleidungsstücken wie zum Beispiel Unterbekleidungs- oder Sportbekleidungsstücke, beispielsweise Büstenhalter, aber auch Oberbekleidungsstücke. Des Weiteren können mit dem erfindungsgemäßen Verfahren auch Bandagen wie zum Beispiel Gelenkbandagen, beispielsweise Knie- oder Ellbogenbandagen hergestellt werden.

Bei dem Verfahren wird eine Stofflage mit einer Kombination bzw. einem Verbund von Elastomer und Verstärkungsmaterial verbunden. Diese Kombination wird erzeugt, indem auf eine Elastomerschicht flüssiges oder weiches und verformbares Verstärkungsmaterial aufgetragen wird, das nach dem Auftragen aushärtet. Das Verstärkungsmaterial besitzt eine höhere Festigkeit als das Elastomer, so dass man auf diese Weise einen Verbund von dem Elastomer und dem Verstärkungsmaterial erhält, der eine erheblich höhere Zugfestigkeit aufweist als das Elastomer ohne Verstärkungsmaterial. Auf das Einbringen von Verstärkungselementen wie zum Beispiel Metallstäbe oder Federn kann verzichtet werden.

Die Elastomerschicht und das Verstärkungsmaterial werden über jeweils eine Düse aufgetragen. Die Position jeder Düse ist in allen drei Raumrichtungen einstellbar, so dass jede Position auf dem Bekleidungsstück oder der Bandage angefahren werden kann. Der Auftrag des Elastomers bzw. des Verstärkungsmaterials auf dem Bekleidungsstück oder der Bandage kann über die Positionsveränderung der Düse entlang einer vorgegebenen Bahn erfolgen und beispielsweise streifenförmig durchgeführt werden. Die Positionseinstellung jeder Düse erfolgt über Stellsignale eines Steuergeräts, wobei die Stellsignale auf der Grundlage einer digitalen Vorlage erzeugt werden können.

Auch die Menge des über die jeweilige Düse austretenden Elastomermaterials und Verstärkungsmaterials kann veränderlich eingestellt werden, insbesondere ebenfalls über Stellsignale des Steuergeräts auf der Grundlage der digitalen Vorlage.

Die Düsen für den Auftrag des Elastomermaterials und des Verstärkungsmaterials können jeweils mit einer eigenen, separaten Verstellmechanik ausgestattet sein. Es ist aber auch möglich, für beide Düsen eine gemeinsame Verstellmechanik vorzusehen, um zunächst die Düse für den Auftrag des Elastomers und anschließend die Düse für den Auftrag des Verstärkungsmaterials mit der gleichen Verstellmechanik anzusteuern. Gegebenenfalls kann danach eine weitere Schicht Elastomer über die entsprechende Düse aufgetragen werden.

Das Verstärkungsmaterial kann in einer Schicht oder gegebenenfalls in mehreren Schichten aufgetragen werden, was es ermöglicht, eine von dem Verstärkungsmaterial gebildete Verstärkungsraupe im Hinblick auf Höhe oder Breite (bezogen auf ihre Längsachse) und/oder Form beliebig zu gestalten. Die mindestens zwei übereinanderliegenden Schichten des Verstärkungsmaterials können sich in ihrer Breite und/oder Höhe unterscheiden, wobei auch Schichten gleicher Breite und/oder Höhe des Verstärkungsmaterials möglich sind.

Die Verstärkungsraupe, gebildet von dem ausgehärteten Verstärkungsmaterial, weist bezüglich ihrer Längsachse einen symmetrischen oder einen nicht-symmetrischen Querschnitt auf, der zum Beispiel in Richtung des Randbereichs des Bekleidungsstücks oder der Bandage reduziert ist.

Bei dem Verstärkungsmaterial handelt es sich beispielsweise um einen Kunststoff, der im flüssigen oder weichen, verformbaren Zustand auf die Elastomerschicht aufgebracht wird. Nach dem Aushärten besitzt das Verstärkungsmaterial eine höhere Zugfestigkeit als das Elastomer, so dass entsprechend auch der Verbund von Elastomer und Verstärkungsmaterial die gewünschte höhere Zugfestigkeit besitzt.

Da das Verstärkungsmaterial im flüssigen oder weichen, verformbaren Zustand aufgetragen wird, bestehen hinsichtlich der Kontur des Verbundes bzw. der Kombination von dem Elastomer und dem Verstärkungsmaterial keine Einschränkungen. Das Elastomer wird beispielsweise in Bahnen aufgetragen, das Verstärkungsmaterial kann in der Weise auf die bahnenförmige Elastomerschicht aufgebracht werden, dass ein seitlicher Abstand zu den seitlichen Randbereichen der Elastomerschicht besteht. Die bahnenförmige Elastomerschicht kann geradlinig oder gekrümmt, gegebenenfalls auch geknickt verlaufen, wobei dieser Verlauf auch von dem aufzutragenden Verstärkungsmaterial problemlos gefolgt werden kann.

Ein weiterer Vorteil liegt darin, dass die Menge des auf die Elastomerschicht aufzubringenden Verstärkungsmaterials bedarfsweise angepasst werden kann. So ist es beispielsweise möglich, entlang der Elastomerschicht an verschiedenen Stellen unterschiedlich viel Verstärkungsmaterial aufzutragen. Des Weiteren ist es auch möglich, eine Bahn aus Verstärkungsmaterial mit gleichbleibender Dicke bzw. gleichbleibendem Durchmesser aufzubringen. Die Bahn aus Verstärkungsmaterial auf der Elastomerschicht ist vorteilhafterweise durchgehend ausgebildet.

Der Auftrag des Verstärkungsmaterials kann in einer durchgehenden geradlinigen oder gekrümmt, gegebenenfalls geknickt verlaufenden Bahn erfolgen oder, in einer weiteren vorteilhaften Ausführung, in einem regellosen, jedoch durchgehenden Auftrag, bei dem sich das Verstärkungsmaterial beispielsweise in einer Schlangenlinie mit seitlichen und/oder vertikalen Auswölbungen zur Auftragungsrichtung erstreckt. Über die schlangenförmigen Linie wird eine zusätzliche Federwirkung im Verstärkungsmaterial erreicht.

Das Verstärkungsmaterial ist vorteilhafterweise vollständig von dem Elastomer umhüllt. Hierdurch wird der Tragekomfort verbessert, da das Verstärkungsmaterial nicht unmittelbar in Kontakt mit der Haut des Trägers gelangen kann.

Die Umhüllung des Verstärkungsmaterials mit Elastomer kann dadurch erfolgen, dass während des Auftragens das Verstärkungsmaterial in das weiche Elastomer der Elastomerschicht eintaucht und innerhalb der Elastomerschicht aushärtet. Gemäß einer vorteilhaften Ausführung kann es aber auch zweckmäßig sein, auf die erste Elastomerschicht, auf die das Verstärkungsmaterial aufgetragen wird, in einem sich anschließenden Schritt eine zweite Elastomerschicht aufzubringen, so dass das Verstärkungsmaterial von der zweiten Elastomerschicht abgedeckt wird. Die erste und die zweite Elastomerschicht bestehen bevorzugt aus dem gleichen Elastomer, das sich zu einer das Verstärkungsmaterial umhüllenden Schicht verbindet. Auf diese Weise ist das Verstärkungsmaterial rundum von Elastomer umhüllt.

Gemäß noch einer weiteren zweckmäßigen Ausführung wird die Elastomerschicht, die Träger des Verstärkungsmaterials ist, unmittelbar auf die Stofflage aufgetragen. Die Stofflage selbst weist, in bevorzugter Ausführung, eine Eigenelastizität bzw. Dehnbarkeit auf, die vorteilhafterweise höher ist als die Dehnbarkeit des ausgehärteten Verstärkungsmaterials. Hierdurch ist sichergestellt, dass die Zugkräfte zumindest zu einem wesentlichen Anteil von dem Verstärkungsmaterial im Elastomer aufgenommen werden. Zugleich verbindet sich das Elastomer mit der Stofflage, so dass eine feste Verbindung zwischen Stofflage und Elastomer gegeben ist. Das im Elastomer eingebettete Verstärkungsmaterial kann zusätzlich mit der Stofflage verbunden werden.

Alternativ zum Auftragen auf eine Stofflage kann die Kombination bzw. der Verbund von Elastomer und Verstärkungsmaterial auch dadurch erzeugt werden, dass eine Elastomerschicht in ein Formteil eingebracht und das Verstärkungsmaterial auf die Elastomerschicht im Formteil aufgebracht wird. Nach dem Aushärten erhält man auf diese Weise eine zusammenhängende Anordnung, die von der Kombination von Elastomer und Verstärkungsmaterial gebildet wird und anschließend mit der Stofflage verbunden werden kann. Die Verbindung erfolgt beispielsweise durch Verkleben, indem zum Beispiel das Elastomer des Verbundes mit der Stofflage verklebt wird, oder auf mechanische Weise, indem das Elastomer oder das Verstärkungsmaterial mit der Stofflage verbunden wird, beispielsweise durch Aufnähen oder Verclipsen oder dergleichen.

Sowohl bei einem Auftragen unmittelbar auf die Stofflage als auch bei einer Herstellung in einem Formteil ist es zweckmäßig, dass auf die erste Elastomerschicht nach dem Auftragen des Verstärkungsmaterials eine zweite Elastomerschicht aufgebracht wird, so dass das Verstärkungsmaterial vollständig innerhalb des Elastomers eingebettet ist.

Gemäß einer weiteren zweckmäßigen Ausführung wird das mit dem Verstärkungsmaterial verstärkte Elastomer mit einem Beflockungsmaterial versehen, beispielsweise mit natürlichen, halbsynthetischen oder synthetischen Fasern bzw. Flocken. Die Beflockung kann im noch nicht vollständig ausgehärteten Zustand des Elastomers aufgebracht werden, so dass nach dem Trocknen das Beflockungsmaterial mit dem Elastomer verklebt ist. Die Beflockung erhöht den Tragekomfort und ist außerdem in der Lage, Feuchtigkeit wie zum Beispiel Schweiß aufzunehmen.

Als Elastomer wird beispielsweise ein thermoplastisches Elastomer verwendet, das bei Erwärmung weich oder flüssig wird und mit dem Abkühlen aushärtet. Dementsprechend wird das Elastomer im erwärmten Zustand als Schicht aufgetragen, wobei das Verstärkungsmaterial im ebenfalls flüssigen oder weichen, verformbaren Zustand vorteilhafterweise auf das noch weiche Elastomer aufgebracht wird.

Das Verstärkungsmaterial besitzt vorzugsweise einen höheren Schmelzpunkt als das Elastomer, so dass mit dem Auftragen das Verstärkungsmaterial das gegebenenfalls teilweise oder vollständig ausgehärtete Elastomer anschmilzt und das Verstärkungsmaterial teilweise in die Elastomerschicht eindringt.

Es sind sowohl Ausführungen möglich, bei denen das Verstärkungsmaterial auf das noch weiche Elastomer aufgetragen wird, als auch Ausführungen, bei denen zunächst die Elastomerschicht aushärtet und erst anschließend das Verstärkungsmaterial aufgetragen wird.

Sofern eine zweite Elastomerschicht aufgebracht wird, erfolgt dies entweder erst nach dem Aushärten des Verstärkungsmaterials oder, in alternativer Ausführung, im noch nicht vollständig ausgehärteten Zustand des Verstärkungsmaterials.

Als Verstärkungsmaterial kommt insbesondere ein Kunststoffmaterial in Betracht, beispielsweise PLA (Polylactid) oder ABS (Acrylnitril-Butadien-Styrol-Copolymerisat). Der Schmelzpunkt des Kunststoffmaterials liegt beispielsweise bei 180° oder 220°.

Das Auftragen des Kunststoffmaterials erfolgt beispielsweise mithilfe eines 3D-Druckkopfes, der es erlaubt, das Kunststoffmaterial in einer gewünschten dreidimensionalen Weise aufzubringen, um beispielsweise über die Länge des Auftrags die Breite und/oder Höhe zu variieren. Dies ermöglicht es, beispielsweise an stärker belasteten Stellen einen breiteren und/oder höheren Auftrag aufzubringen und an weniger belasteten Stellen einen schmaleren und/oder niedrigeren Auftrag aufzubringen. Mithilfe des 3D-Druckkopfes wird das Verstärkungsmaterial schichtweise aufgetragen, bis die gewünschte endgültige Form des Verstärkungsmaterials auf der Elastomerschicht erreicht ist.

Das Auftragen des Kunststoffmaterials kann auch mithilfe einer Spritzvorrichtung, zum Beispiel mit einer Spritzpistole durchgeführt werden.

Gemäß einer weiteren zweckmäßigen Ausführung werden auf die Stofflage mehrere bahnenförmige Elastomerschichten aufgebracht, die ohne Verstärkungsmaterial versehen sind. Nach dem Aufbringen der bahnenförmigen Elastomerschichten wird die Stofflage anschließend innerhalb der Elastomerschichten entsprechend der Randkontur des zu erzeugenden Bekleidungsstücks bzw. der Bandage ausgeschnitten. Dies ermöglicht es, ein Bekleidungsstück bzw. eine Bandage herzustellen, deren randseitige Kontur mit der Elastomerschicht verstärkt ist, wohingegen die innen liegenden, zur randseitigen Kontur beabstandeten Abschnitte der Stofflage ohne durchgehende, flächige Beschichtung mit Elastomer versehen sind und dementsprechend ein verbesssertes Tragegefühl gewährleisten. Die randseitige Elastomerschicht weist eine hohe Elastizität und Tragkraft auf.

Die bahnenförmigen Elastomerschichten können entweder auf nur eine einzige Stofflage aufgebracht werden oder zur Verbindung von zwei Stofflagen dienen, indem zunächst eine Elastomerschicht auf eine Stofflage aufgebracht wird und anschließend auf die erste Stofflage eine zweite Stofflage aufgelegt wird.

Verschiedene bahnenförmige Elastomerschichten ohne Verstärkungsmaterial auf die Stofflage aufzubringen, kann gegebenenfalls mit einer Elastomerschicht mit darin eingebrachtem Verstärkungsmaterial kombiniert werden. Zum Beispiel kann es zweckmäßig sein, bei einem Bekleidungsstück einen Teil der Randkontur aus einer bahnenförmigen Elastomerschicht ohne Verstärkungsmaterial zu bilden und einen Teil aus einer Elastomerschicht mit Verstärkungsmaterial. Beispielsweise können im Falle eines Büstenhalters die Träger aus einem Verbund von bahnenförmigen Elastomerschichten mit darin eingebrachtem Verstärkungsmaterial gefertigt sein. Auch im Bereich dieser, mit Verstärkungsmaterial versehenen Elastomerschichten ist ein Ausschneiden zum Erzeugen der gewünschten Randkultur möglich, wobei der Schnitt vorzugsweise nicht durch das Verstärkungsmaterial, sondern seitlich entlang des Verstärkungsmaterials, jedoch noch innerhalb der Elastomerschicht erfolgt.

Die Elastomerschichten - sowohl mit als auch ohne Verstärkungsmaterial - werden beispielsweise im Siebdruckverfahren aufgetragen, wobei grundsätzlich auch andere Auftragungstechniken in Betracht kommen, beispielsweise Hochdruck, Tiefdruck, Rouleaux (Tiefdruckverfahren, bei dem gravierte Druckwalzen das Elastomer auf den Stoff auftragen), Sprühverfahren, Aufbringen über Düsen oder dergleichen. Die Elastomerschichten können auch über ein Dosiersystem aufgetragen werden.

Weitere Vorteile und zweckmäßige Ausführungen sind den weiteren Ansprüchen, der Figurenbeschreibung und den Zeichnungen zu entnehmen. Es zeigen:
- Fig. 1: eine schematische Ansicht eines Büstenhalters, dessen Träger aus einem Verbund von Elastomer, das auf eine Stofflage aufgebracht ist, und Verstärkungsmaterial bestehen,
- Fig. 2: in Seitenansicht die Kombination von Elastomer und Verstärkungsmaterial in verschiedenen Stufen der Herstellung,
- Fig. 3: in Draufsicht die Kombination von Elastomer und Verstärkungsmaterial.

In den Figuren sind gleiche Bauteile mit gleichen Bezugszeichen versehen.

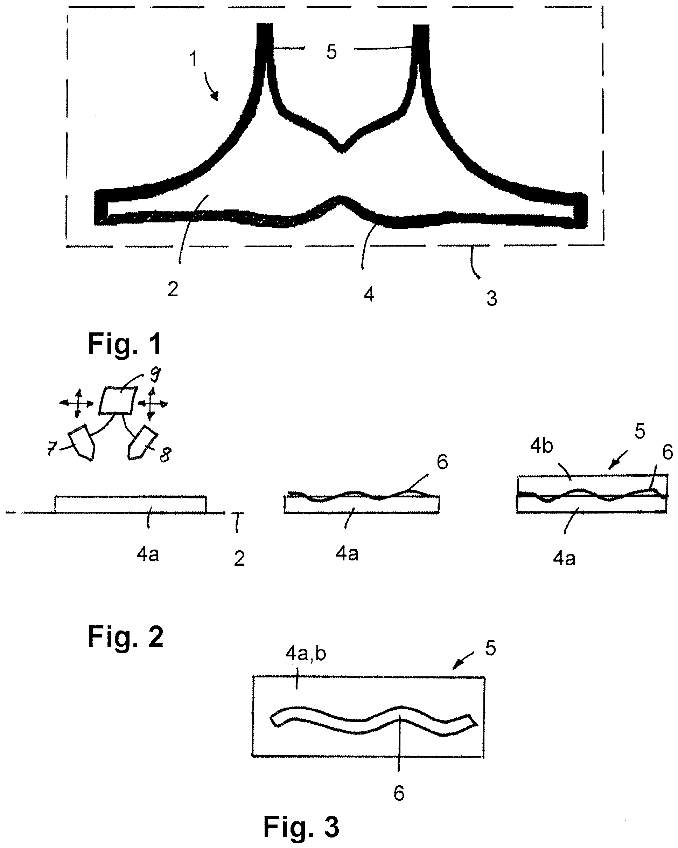

In Fig. 1 ist in schematischer Weise als Bekleidungsstück 1 ein Büstenhalter dargestellt, der eine oder mehrere Stofflagen 2 aufweist und im Randbereich von einer Elastomerschicht 4 eingefasst ist. Die Stofflage 2 bzw. die Stofflagen weisen vor dem Zuschnitt ein beispielsweise rechteckförmiges Grundformat 3 auf, innerhalb dem das Elastomer in den bahnenförmigen Schichten 4 so aufgetragen wird, dass die Randkontur des herzustellenden Büstenhalters gebildet wird. Die Elastomerschicht 4 weist eine Breite von beispielsweise 2 mm bis 20 mm auf. Nach dem Austrocknen des Elastomers erfolgt der Zuschnitt, indem innerhalb der die Randkontur bildenden, bahnenförmigen Elastomerschicht 4 geschnitten wird.

Auch die Träger 5 sind von einer die Randkontur bildenden Elastomerschicht 4 gebildet. Allerdings ist im Bereich der Träger 5 in das Elastomer ein Verstärkungsmaterial 6 eingebracht (Fig. 2, 3), das den Trägern 5 eine deutlich höhere Zugfestigkeit verleiht. Das Verstärkungsmaterial 6 besteht insbesondere aus einem Kunststoffmaterial, beispielsweise PLA oder ABS, und ist vollständig von dem Elastomermaterial umhüllt.

Fig. 2 zeigt drei verschiedene Schritte bei der Herstellung des Trägers 5 aus der Kombination von Elastomerschicht 4 und Verstärkungsmaterial 6. Wie im linken Bild von Fig. 2 zu erkennen, wird zunächst in einem ersten Schritt eine erste Elastomerschicht 4 auf die Stofflage 2 aufgebracht. Hierzu wird das Elastomer vorteilhafterweise erwärmt, so dass das Elastomer verformbar, insbesondere streichfähig ist.

In einem zweiten Schritt, der in dem mittleren Bild von Fig. 2 dargestellt ist, wird anschließend das Verstärkungsmaterial 6 im flüssigen oder zumindest weichen, verformbaren Zustand auf die erste Elastomerschicht 4a aufgetragen. Das Auftragen des Kunststoffmaterials erfolgt in einer Schicht oder in mehreren Schichten beispielsweise mithilfe eines 3D-Druckkopfes, wobei es auch möglich ist, das Kunststoffmaterial beispielsweise mithilfe einer Spritzpistole oder einer beheizten Düse aufzutragen. Das Verstärkungsmaterial 6 weist eine verhältnismäßig hohe Temperatur von beispielsweise mindestens 180° C auf, um sicherzustellen, dass sich das Verstärkungsmaterial im flüssigen oder zumindest weichen, verformbaren Zustand befindet. Das Verstärkungsmaterial 6 kann gegebenenfalls, wie in den Fig. angedeutet, schlangenlinienförmig aufgebracht werden, und zwar sowohl orthogonal zur Ebene der Stofflage 2 als auch innerhalb der Ebene der Stofflage 2 und quer zur Längserstreckung des Trägers 5.

In einem dritten, sich anschließenden Schritt, der im rechten Bild von Fig. 2 dargestellt ist, wird auf die untere Elastomerschicht 4a eine zweite, obere Elastomerschicht 4b aufgetragen, die das Verstärkungsmaterial 6 vollständig umhüllt. Der Auftrag der zweiten Elastomerschicht 4b erfolgt vorzugsweise nach dem Aushärten des Verstärkungsmaterials 6.

Im linken Bild von Fig. 2 sind zwei Düsen 7, 8 dargestellt, über die die Elastomerschicht 4 und das Verstärkungsmaterial 6 auf die Stofflage 2 aufgetragen werden. Die Düsen 7, 8 können über eine Verstellmechanik in alle drei Raumrichtungen verstellt werden und beliebige Positionen über der Stofflage einnehmen. Während des Auftragens der Elastomerschichten 4a und 4b über die Düse 7 und des Verstärkungsmaterials 6 über die Düse 8 werden die Düsen entlang der vorgesehenen Bahnen über die Stofflage 2 bewegt; hierbei tritt das Elastomermaterial bzw. das Verstärkungsmaterial aus der betreffenden Düse aus, wobei die Austrittsmenge des Elastomer- bzw-Verstärkungsmaterials in Abhängigkeit von der aktuellen Düsenposition gesteuert wird.

Die Positionseinstellung jeder Düse 7, 8 sowie die Menge des über die jeweilige 7, 8 Düse austretenden Elastomer- bzw-Verstärkungsmaterials erfolgen über Stellsignale eines Steuergeräts 9, wobei die Stellsignale auf der Grundlage einer digitalen Vorlage erzeugt werden.

Fig. 3 zeigt die Draufsicht auf den Träger 5 mit dem innerhalb des Elastomers eingebetteten Verstärkungsmaterial 6, das zu den seitlichen Rändern - quer zur Längserstreckung des Verstärkungsmaterials 6 gesehen - jeweils einen Abstand aufweist.

Alternativ zu der dargestellten schlangenlinienförmigen Ausführung des Verstärkungsmaterials 6 kommt auch eine geradlinige Ausführung oder eine gekrümmte, gegebenenfalls geknickte Ausführung des Verstärkungsmaterials 6 in Betracht. Vorzugsweise folgt der Verlauf des Verstärkungsmaterials 6 dem Verlauf der Elastomerschicht 4. Es kann vorteilhaft sein, das Verstärkungsmaterial 6 im Bereich eines Endes mit der Stofflage unmittelbar zu verbinden, beispielsweise auf mechanische Weise wie zum Beispiel durch Vernähen, um eine feste Verbindung zwischen Verstärkungsmaterial 6 und Stofflage 2 zu schaffen.

## Patentansprüche

1. Verfahren zur Herstellung von Bekleidungsstücken (1) oder Bandagen, mit einer Stofflage (2), die mit einem Verbund von Elastomer und Verstärkungsmaterial (6) verbunden wird,
**dadurch gekennzeichnet,**
**dass** der Verbund erzeugt wird, indem auf eine Elastomerschicht (4) das Verstärkungsmaterial (6) im flüssigen oder weichen, verformbaren Zustand aufgetragen wird, das nach dem Auftragen aushärtet, wobei die Elastomerschicht (4) und das Verstärkungsmaterial (6) über jeweils eine Düse (7,8) aufgetragen werden, deren Position in drei Raumrichtungen einstellbar ist, wobei die Menge des über die jeweilige Düse (7, 8) austretenden Elastomermaterials und Verstärkungsmaterials (6) jeweils einstellbar ist.

2. Verfahren nach Anspruch 1,
**dadurch gekennzeichnet,**
**dass** das Verstärkungsmaterial (6) in mehreren Schichten aufgetragen wird.

3. Verfahren nach Anspruch 2,
**dadurch gekennzeichnet,**
**dass** mindestens zwei übereinanderliegende Schichten des Verstärkungsmaterials (6) sich in ihrer Breite und/oder Höhe unterscheiden.

4. Verfahren nach einem der Ansprüche 1 bis 3,
**dadurch gekennzeichnet,**
**dass** die Düse, über die das Verstärkungsmaterial (6) aufgetragen wird, als ein 3D-Druckkopf ausgebildet ist.

5. Verfahren nach einem der Ansprüche 1 bis 4,
**dadurch gekennzeichnet,**
**dass** das Verstärkungsmaterial (6) einen höheren Schmelzpunkt als das Elastomer besitzt.

6. Verfahren nach einem der Ansprüche 1 bis 5,
**dadurch gekennzeichnet,**
**dass** das Verstärkungsmaterial (6) vollständig von der Elastomerschicht (4) umhüllt ist.

7. Verfahren nach einem der Ansprüche 1 bis 6,
**dadurch gekennzeichnet,**
**dass** auf die Elastomerschicht (4) eine das Verstärkungsmaterial (6) einschließende zweite Elastomerschicht (4) aufgetragen wird.

8. Verfahren nach einem der Ansprüche 1 bis 7,
**dadurch gekennzeichnet,**
**dass** die Elastomerschicht (4) unmittelbar auf die Stofflage (2) aufgetragen wird.

9. Verfahren nach einem der Ansprüche 1 bis 8,
**dadurch gekennzeichnet,**
**dass** die Elastomerschicht (4) in ein Formteil eingebracht und das Verstärkungsmaterial (6) auf die Elastomerschicht (4) im Formteil aufgebracht wird.

10. Verfahren nach Anspruch 9,
**dadurch gekennzeichnet,**
**dass** der Verbund von Elastomer und Verstärkungsmaterial (6) nach dem Aushärten mit der Stofflage (2) verbunden wird.

11. Verfahren nach einem der Ansprüche 1 bis 10,
**dadurch gekennzeichnet,**
**dass** mehrere, bahnenförmige Elastomerschichten (4) mit oder ohne Verstärkungsmaterial (6) auf die Stofflage (2) aufgebracht werden, wobei die Stofflage (2) anschließend innerhalb der bahnenförmigen Elastomerschichten (4) entsprechend der Randkontur des Bekleidungsstücks (1) bzw. der Bandage ausgeschnitten wird.

12. Verfahren nach Anspruch 11,
**dadurch gekennzeichnet,**
**dass** die bahnenförmigen Elastomerschichten (4) auf genau eine Stofflage (2) aufgebracht werden.

13. Verfahren nach Anspruch 11,
**dadurch gekennzeichnet,**
**dass** die bahnenförmigen Elastomerschichten (4) auf eine Stofflage (2) aufgebracht werden und anschließend eine zweite Stofflage (2) aufgelegt wird.

14. Verfahren nach einem der Ansprüche 1 bis 13,
**dadurch gekennzeichnet,**
**dass** sowohl Elastomerschichten (4) mit Verstärkungsmaterial (6) als auch Elastomerschichten (4) ohne Verstärkungsmaterial (6) auf die Stofflage (2) aufgebracht werden.

15. Verfahren nach einem der Ansprüche 1 bis 14,
**dadurch gekennzeichnet,**
**dass** das mit oder ohne Verstärkungsmaterial (6) versehene Elastomer beflockt wird.

16. Verfahren nach einem der Ansprüche 1 bis 15,
**dadurch gekennzeichnet,**
**dass** als Verstärkungsmaterial (6) ein Kunststoffmaterial wie zum Beispiel PLA (Polylactid) oder ABS (Acrylnitril-Butadien-Styrol-Copolymerisat) verwendet wird.

17. Verfahren nach einem der Ansprüche 1 bis 16,
**dadurch gekennzeichnet,**
**dass** ein thermoplastisches Elastomer verwendet wird.

18. Verfahren nach einem der Ansprüche 1 bis 17,
**dadurch gekennzeichnet,**
**dass** als Elastomer ein Silikon verwendet wird.

## Claims

1. Method for production of apparel pieces (1) or bandages, with a fabric ply (2) to be bonded to a composite combining an elastomer and a reinforcing material (6),
**characterized in that**
the composite is formed by an elastomer layer (4) having the reinforcing material (6) applied to it while the latter is in the liquid or soft, deformable state and hardens after application, wherein the elastomer layer (4) and the reinforcing material (6) are each applied via a nozzle (7, 8) whose position is adjustable in three spatial directions, wherein the amount of the elastomer material and of the reinforcing material (6) emerging via the respective nozzle (7, 8) is adjustable in both cases.

2. Method according to Claim 1,
**characterized in that**
the reinforcing material (6) is applied in two or more layers.

3. Method according to Claim 2,
**characterized in that**
at least two superposed layers of the reinforcing material (6) differ in width and/or height.

4. Method according to any one of Claims 1 to 3,
**characterized in that**
the nozzle via which the reinforcing material (6) is applied is configured as a 3D printing head.

5. Method according to any one of Claims 1 to 4,
**characterized in that**
the reinforcing material (6) has a higher melting point than the elastomer.

6. Method according to any one of Claims 1 to 5,
**characterized in that**
the elastomer layer (4) fully envelops the reinforcing material (6).

7. Method according to any one of Claims 1 to 6,
**characterized in that**
the elastomer layer (4) has applied to it a second elastomer layer (4) which encloses the reinforcing material (6).

8. Method according to any one of Claims 1 to 7,
**characterized in that**
the elastomer layer (4) is directly applied to the fabric ply (2).

9. Method according to any one of Claims 1 to 8,
**characterized in that**
the elastomer layer (4) is incorporated in a moulding and the reinforcing material (6) is deposited onto the elastomer layer (4) in the moulding.

10. Method according to Claim 9,
**characterized in that**
the composite combining an elastomer and a reinforcing material (6) is bonded to the fabric ply (2) after hardening.

11. Method according to any one of Claims 1 to 10,
**characterized in that**
two or more web-shaped elastomer layers (4) with or without reinforcing material (6) are deposited onto the fabric ply (2), wherein the fabric ply (2) is subsequently cut out within the web-shaped elastomer layers (4) according to the edge contour of the apparel piece (1) or bandage.

12. Method according to Claim 11,
**characterized in that**
the web-shaped elastomer layers (4) are deposited onto one fabric ply (2).

13. Method according to Claim 11,
**characterized in that**
the web-shaped elastomer layers (4) are deposited onto one fabric ply (2) and then a second fabric ply (2) is laid on top.

14. Method according to any one of Claims 1 to 13,
**characterized in that**
not only elastomer layers (4) with reinforcing material (6) but also elastomer layers (4) without reinforcing material (6) are deposited onto the fabric ply (2).

15. Method according to any one of Claims 1 to 14,
**characterized in that**
an elastomer with or without reinforcing material (6) is flocked.

16. Method according to any one of Claims 1 to 15,
**characterized in that**
a plastics material such as, for example, PLA (polylactide) or ABS (acrylonitrile-butadiene-styrene copolymer) is used as reinforcing material (6).

17. Method according to any one of Claims 1 to 16,
**characterized in that**
a thermoplastic elastomer is used.

18. Method according to any one of Claims 1 to 17,
**characterized in that**
a silicone is used as elastomer.

## Revendications

1. Procédé de fabrication d'articles vestimentaires (1) ou de bandages, comprenant une couche de tissu (2), qui est reliée avec un composite d'un élastomère et d'un matériau de renfort (6), **caractérisé en ce que** le composite est formé par application sur une couche d'élastomère (4) du matériau de renfort (6) à l'état liquide ou mou, façonnable, qui durcit après l'application, la couche d'élastomère (4) et le matériau de renfort (6) étant chacun appliqués par une buse (7, 8), dont la position est ajustable dans les trois directions de l'espace, la quantité du matériau élastomère et du matériau de renfort (6) sortant par la buse respective (7, 8) étant à chaque fois ajustable.

2. Procédé selon la revendication 1, **caractérisé en ce que** le matériau de renfort (6) est appliqué en plusieurs couches.

3. Procédé selon la revendication 2, **caractérisé en ce qu'**au moins deux couches superposées du matériau de renfort (6) diffèrent l'une de l'autre par leur largeur et/ou leur hauteur.

4. Procédé selon l'une quelconque des revendications 1 à 3, **caractérisé en ce que** la buse par laquelle le matériau de renfort (6) est appliqué est configurée sous la forme d'une tête d'impression 3D.

5. Procédé selon l'une quelconque des revendications 1 à 4, **caractérisé en ce que** le matériau de renfort (6) présente un point de fusion plus élevé que l'élastomère.

6. Procédé selon l'une quelconque des revendications 1 à 5, **caractérisé en ce que** le matériau de renfort (6) est entièrement enrobé par la couche d'élastomère (4).

7. Procédé selon l'une quelconque des revendications 1 à 6, **caractérisé en ce qu'**une deuxième couche d'élastomère (4) incluant le matériau de renfort (6) est appliquée sur la couche d'élastomère (4).

8. Procédé selon l'une quelconque des revendications 1 à 7, **caractérisé en ce que** la couche d'élastomère (4) est appliquée directement sur la couche de tissu (2).

9. Procédé selon l'une quelconque des revendications 1 à 8, **caractérisé en ce que** la couche d'élastomère (4) est introduite dans une pièce moulée et le matériau de renfort (6) est appliqué sur la couche d'élastomère (4) dans la pièce moulée.

10. Procédé selon la revendication 9, **caractérisé en ce que** le composite d'un élastomère et d'un matériau de renfort (6) est relié avec la couche de tissu (2) après le durcissement.

11. Procédé selon l'une quelconque des revendications 1 à 10, **caractérisé en ce que** plusieurs couches d'élastomère en forme de bande (4) avec ou sans matériau de renfort (6) sont appliquées sur la couche de tissu, la couche de tissu (2) étant ensuite découpée dans les couches d'élastomère en forme de bande (4) selon le contour de bordure de l'article vestimentaire (1) ou du bandage.

12. Procédé selon la revendication 11, **caractérisé en ce que** les couches d'élastomère en forme de bande (4) sont appliquées sur exactement une couche de tissu (2).

13. Procédé selon la revendication 11, **caractérisé en ce que** les couches d'élastomère en forme de bande (4) sont appliquées sur une couche de tissu (2), puis une deuxième couche de tissu (2) est déposée.

14. Procédé selon l'une quelconque des revendications 1 à 13, **caractérisé en ce qu'**aussi bien des couches d'élastomère (4) avec matériau de renfort (6) que des couches d'élastomère (4) sans matériau de renfort (6) sont appliquées sur la couche de tissu (2).

15. Procédé selon l'une quelconque des revendications 1 à 14, **caractérisé en ce que** l'élastomère avec ou sans matériau de renfort (6) est floqué.

16. Procédé selon l'une quelconque des revendications 1 à 15, **caractérisé en ce qu'**un matériau plastique tel que par exemple le PLA (polylactide) ou l'ABS (copolymère acrylonitrile-butadiène-styrène) est utilisé en tant que matériau de renfort (6).

17. Procédé selon l'une quelconque des revendications 1 à 16, **caractérisé en ce qu'**un élastomère thermoplastique est utilisé.

18. Procédé selon l'une quelconque des revendications 1 à 17, **caractérisé en ce qu'**une silicone est utilisée en tant qu'élastomère.
